**Europäisches Patentamt**

(19) **European Patent Office**

**Office européen des brevets**

(11) Veröffentlichungsnummer: **0 211 204**
**B1**

(12) **EUROPÄISCHE PATENTSCHRIFT**

(45) Veröffentlichungstag der Patentschrift:
**10.10.90**

(51) Int. Cl.⁵: **A61K 9/10,** A61K 31/23,
A61K 37/18, A61K 47/00

(21) Anmeldenummer: **86108465.5**

(22) Anmeldetag: **20.06.86**

(54) Verfahren zur Herstellung einer stabilen, wässrigen Fettemulsion und deren Verwendung in parenteral zu verabreichenden Gesamtnährlösungen.

(30) Priorität: **11.07.85 DE 3524788**

(43) Veröffentlichungstag der Anmeldung:
**25.02.87 Patentblatt 87/9**

(45) Bekanntmachung des Hinweises auf die Patenterteilung:
**10.10.90 Patentblatt 90/41**

(84) Benannte Vertragsstaaten:
**AT BE CH DE FR GB IT LI LU NL SE**

(56) Entgegenhaltungen:
**DE-B- 1 792 294**
**DE-B- 1 934 317**
**FR-A- 2 216 926**

**CHEMICAL ABSTRACTS, Band 104, Nr. 26, 30. Juni 1986, Seite 365, Zusammenfassung Nr. 230504n, Columbus, Ohio, US; & JP-A-61 56 124**
**CHEMICAL ABSTRACTS, Band 104, Nr. 8, 24. Februar 1986, Seite 368, Zusammenfassung Nr. 56448k, Columbus, Ohio, US; & JP-A-60 199 814**

(73) Patentinhaber: **LEOPOLD PHARMA GESELLSCHAFT m.b.H., Hafnerstrasse 36, A-8055 Graz(AT)**

(84) Benannte Vertragsstaaten: **BE CH DE FR GB IT LI LU NL SE AT**

(72) Erfinder: **Groke, Karl, Dr., A-8063 Eggersdorf 86(AT)**
Erfinder: **Polzer, Josef, Grottenhofstrasse 38/20, A-8053 Graz(AT)**

(74) Vertreter: **Karau, Wolfgang, Dr. et al, BASF Aktiengesellschaft Patentabteilung ZSP - C 6 Carl-Bosch-Strasse 38, D-6700 Ludwigshafen(DE)**

**Beschreibung**

Die vorliegende Erfindung betrifft ein Verfahren zur Herstellung einer stabilen wäßrigen Fettemulsion durch Emulgieren unter hohem Druck unter Einsatz von Phospholipiden, insbesondere Lezithinen, als Emulgator und deren Verwendung in zur parenteralen Verabreichung bestimmten Gesamtnährlösungen.

Seit längerer Zeit sind Bestrebungen im Gange bei der parenteralen Ernährung, insbesondere bei Patienten in kataboler Stoffwechselsituation, neben Kohlehydraten und Aminosäuren Fettemulsionen zur Deckung des Energiebedarfes heranzuziehen. Hierbei war es immer schon das Ziel, solche Fettemulsionen, die bevorzugt Öl- in Wasser-Emulsionen sind, mit den anderen zu verabreichenden Nährstoffen zu einer Gesamtnährlösung zu kombinieren. Diesem Bestreben steht im Wege, daß Fettemulsionen, die in ausreichender Tröpfchenfeinheit und Stabilität aus Öl unter Verwendung von Emulgatoren, insbesondere Lezithinen, herstellbar sind, in Gegenwart der anderen Nährstoffe ihre Stabilität einbüßen bzw. zum Brechen neigen. Es liegen bereits einige Versuche vor, diese Schwierigkeiten zu lösen.

So werden in der DE-PS 1,249.454 wäßrige, 5 - 50 Gew.%ige Fettemulsionen mit einer Tröpfchengröße unter 4 µm, sogar bis zu 1 µm, beschrieben, die mit Hilfe von Eilezithin als Emulgator erhalten werden. Als wäßrige Phase kann hierbei u. a. 5 %ige Glucoselösung oder 2,5 %ige Glycerinlösung, die der Emulsion gegenüber reinem Wasser eine bessere Stabilität verleihen, dienen. Gemäß DE-AS 1,792.294 ist es möglich, Nährlösungen die neben einer Emulsion von Sojaöl in Wasser mit Sojaphosphatiden als Emulgator nicht nur alle essentiellen Aminosäuren, sondern auch einige nicht essentielle Aminosäuren sowie Zuckeralkohole enthalten, herzustellen, wenn auf die Einverleibung der stark polaren Aminosäure Arginin, verzichtet wird und auch möglichst weder Glutaminsäure noch Asparaginsäure zugesetzt werden. Auf diese Weise ist es möglich, Nährlösungen mit einem Fettgehalt von 1 bis 15 % und einem Aminosäuregehalt von 1 bis 10 % mit einer Teilchengöße um 1 µm zu erhalten, die stabil sein sollen. Die Einverleibung der ebenfalls für den Organismus sehr wichtigen Elektrolyte muß bei diesen Lösungen gemäß DE-AS 1,792.294 unterbleiben, da diese das Brechen der Emulsion innerhalb kürzester Zeit bewirken würden.

Die Herstellung solcher Emulsionen erfolgt gemäß DE-AS 1,792.294 durch Einmischen von Öl und Emulgator in eine wäßrige Aminosäurelösung in einem schnellaufenden Mischer und anschließende Emulgierung unter einem Druck von etwa 400 bar, worauf die erhaltene Lösung hitzesterilisiert wird.

Gemäß DE-AS 1,934.317 können Lösungen mit höherer Lagerstabilität erhalten werden, wenn die Aminosäurelösung und die Fettemulsion getrennt hergestellt und vorzugsweise auch getrennt sterilisiert werden und anschließend die Mischung der Bestandteile vorgenommen wird.

Gemäß DE-PS 2,406.621 wird es als Mangel dieser vorbekannten Lösungen angesehen, daß sie das zur Blutammoniakentgiftung wichtige Arginin sowie Glutaminsäure und Asparaginsäure nicht enthalten können. Werden diese Aminosäuren jedoch Fettemulsionen zugesetzt, was gemäß B. Helwig, Moderne Arzneimittel, 1972, Seiten 1177 und 1179 empfohlen wird, so enthalten diese Lösungen gemäß Ausführungen in der DE-PS 2,406.621 trotz eines stabilisierenden Kohlehydratgehaltes und einer Partikelgröße von vorzugsweise unter 0,1 µm zahlreiche weitaus größere Tröpfchen, beispielsweise bis zu 12 µm, die für die intravenöse Applikation nachteilig sind. Ferner ist die Stabilität dieser Lösungen für den praktischen Gebrauch zu gering.

Zur Abhilfe wird in der DE-PS 2,406.621 der Zusatz von 0,02 bis 3 g einer Fettsäure mit 11 bis 20 C-Atomen oder eines basischen Aminosäuresalzes derselben pro g an eingesetztem Ei-Phospholipid empfohlen, wobei solche Lösun gen trotz Gehalt an den genannten stark polaren Aminosäuren und 20 Minuten dauernder Sterilisation bei 120°C eine Tröpfchengröße unter 0,5 µm beibehalten. Ein Elektrolytgehalt ist jedoch auch bei diesen Lösungen nicht vorgesehen.

Andererseits ist bekannt, daß Ubidecarenon interessante pharmazeutische Wirkungen entfaltet, sodaß es u. a. auch schon intravenös verabreicht wurde. So wird z. B. in der DE-OS 3,405.581 die Verabreichung eines Gemisches von Ubidecarenon und Carnitin oder Acetylcarnitin in der Herz- und Gefäßtherapie empfohlen. In wäßriger Lösung unter Anwendung von Lezithin als Emulgator wird es gemäß EPA 0 100 459 verabreicht, wobei diese Lösungen zusätzlich Äthanol und/oder Isopropanol enthalten und die Lezithinmenge in Abhängigkeit von der vorhandenen Menge an Alkoholen zu wählen ist. So darf z. B. die Lezithinmenge nicht mer als 0,1 part per weight betragen, wenn weniger als 50 Teil Äthanol pro Teil fettlöslicher Substanz , u. a. Ubidecarenon, zugegen sind.

Gemäß EP-A 0 132 821 können stabile wäßrige Lösungen von Ubidecarenon mit hydriertem Lezithin als Emulgator erhalten werden, wenn der pH-Wert mittels neutralen Aminosäuren wie Prolin oder Glycin auf 5,5 bis 8 eingestellt wird. Zur Verkürzung der Dispergierzeit durch Ultraschallwirkung wird aber auch hier der Zusatz eines Lösungsmittels wie Äthanol, Propylenglykol, niedermolekulares Polyäthylenglycol oder Glycerin neben einem Gehalt an Sorbit empfohlen.

Überraschenderweise konnte nun gefunden werden, daß es möglich ist, völlig stabile, sterilisierbare Fettemulsionen mit Phospholipiden, insbesondere Lezithinen, als Emulgator zu erhalten, denen nicht nur Zuckeralkohole und sog. neutrale Aminosäuren sondern alle unter medizinischen Gesichtspunkten auszuwählenden Aminosäuren in eben unter diesem Gesichtspunkt zu wählender Menge sowie sogar die nötigen Elektrolyte einverleibt werden können, ohne die Stabilität der Emulsion zu gefährden, wenn zusätzlich zu dem Lezithinemulgator Ubidecarenon als Hilfsemulgator eingesetzt wird. Damit ist es erstmals möglich geworden, fetthaltige Gesamtnährlösungen mit einer allein aus medizinischen Überlegungen heraus gewählten Zusammensetzung zu schaffen, ohne auf eine ev. Stabilitätsbeeinträchtigung Rücksicht nehmen zu müssen.

Gegenstand der vorliegenden Erfindung ist demnach ein Verfahren zur Herstel lung einer stabilen wäßrigen Fettemulsion zur Verwendung in Lösungen zur parenteralen Ernährung, durch Emulgieren des Fettes unter hohem Druck und unter Einsatz von Phospholipiden, insbesondere Lezithinen, als Emulgator, das dadurch gekennzeichnet ist, daß neben dem als Emulgator dienenden Phospholipid Ubidecarenon als Hilfsemulgator in einem Gewichtsverhältnis von Phospholipid zu Ubidecarenon von 150 : 1 bis 7,5 : 1 eingesetzt wird und das Gewicht der beiden Emulgatoren zusammen zum Gewicht der zu emulgierenden Fettmenge im Verhältnis 1 : 7 bis 1 : 30 steht.

Die erfindungsgemäß hergestellte wäßrige Fettemulsion zeichnet sich durch eine gleichmäßige Tröpfchengröße von etwa 1 µm aus, die auch bei längerer Lagerung erhalten bleibt und sich auch dann nicht verändert, wenn weitere, für parenterale Nährlösungen übliche Zusätze darin enthalten sind. Ubidecarenon ist ferner ein Antioxidans, sodaß es gleichzeitig auch das Fett und auch ev. vorhandene, andere oxydationsempfindliche Zusätze wie z. B Aminosäuren gegen Oxydation schützt.

Als Emulgator sind Sojalezithin oder Fraktionen desselben bevorzugt. Es können aber auch Eilezithine, hochgereinigte native Lezithine oder synthetische Phospholipide mit chemisch definierter Zusammensetzung Verwendung finden.

Als Fettkomponente dient in üblicher Weise Öl, wobei Sojaöl bevorzugt ist. Die Gesamtmenge an Emulgator und Ubidecarenon steht zum zu emulgierenden Fett vorzugsweise in einem Gewichtsverhältnis von 1 : 7,5 bis 1 : 15. Als Gewichtsverhältnis von Phospholipid zu Ubicarenon ist ein solches von 30 : 1 bis 10 : 1 besonders bevorzugt.

Die praktische Durchführung des erfindungsgemäßen Verfahrens kann auf die an sich übliche Weise durch Bereitung einer Voremulsion in einem Schnellmischer und anschließende mehrstufige Hochdruckemulgierung bei 400 - 600 bar erfolgen, wobei bei der Herstellung der Voremulsion das Öl in die berechnete Menge Wasser eingetragen wird, die das Phospholipid sowie alle ev. vorgesehenen Zusätze wie Aminosäuren, Zuckeralkohole und dergleichen enthält. Zu beachten ist hierbei, daß das Ubidecarenon im Gemisch mit dem Öl eingebracht wird. Der Vorteil der erfindungsgemäßen Zugabe des Ubidecarenons zeigt sich dabei in einer sehr raschen Bildung der Voremulsion einer gleichmäßigen Tröpfchengröße von 2 bis 3 µm, beispielsweise bei Anwendung von etwa 5000 U/Minute. Zweckmäßigerweise arbeitet man dabei bei Normaldruck, besser aber noch im Vakuum, z. B. bei 0,5 bis 0,6 bar, wobei das vorgelegte wäßrige Gemisch zweckmäßig vorgewärmt wird, vorzugsweise auf eine Temperatur von 70 - 80 °C und bevorzugt mit NaOH oder einer anderen parenteral verträglichen Base auf einen pH-Wert von 7,0 bis 8,5 gebracht wurde. Ferner empfiehlt es sich, das Ubidecarenon in etwas warmem Öl aufzulösen, der Rest der eingesetzten Ölkomponente muß dann nicht auch vorgewärmt werden. Die Anwendung einer $N_2$-Atmosphäre während der Bereitung der Emulsion schützt Bestandteile vor schädlicher $O_2$-Wirkung, die z. B. zum Ranzigwerden des Fettes führen könnte.

Die so erhaltene Voremulsion wird auf Grund des Ubidecarenon-Gehaltes nicht nur rasch erhalten, sie ist auch für die Weiterverarbeitung im Hochdruckemulgator hervorragend geeignet.

Diese wird in der Regel bei 400 - 600 bar im mehreren, z. B. 4 Schritten durchgeführt, im Fall der vorliegenden Erfindung kann sogar mit etwa 400 bar das Auslangen gefunden werden.

Anschließend ist es zweckmäßig, das so erhaltene Produkt durch Filtration, beispielsweise über ein 3 µm Filter, von ev. noch vorliegenden größeren Bestandteilen zu befreien. Die Keimfreimachung kann durch jede übliche Methode, z. B. durch Sterilisation, Tyndallisieren etc. erfolgen.

Ein weiterer Gegenstand der vorliegenden Erfindung ist die Verwendung der erfindungsgemäß hergestellten Fettemulsion in Gesamtnährlösungen, die zur parenteralen Verabreichung bestimmt sind.

Dabei ist es ein besonderer Vorteil der erfindungsgemäß hergestellten Fettemulsion, daß sie Elektrolyte enthalten kann, ohne daß sie in der Stabilität beeinträchtigt wird, es ist daher möglich, mit dem erfindungsgemäßen Verfahren Gesamtnährlösungen mit einem Elektrolytgehalt üblicher Menge und Zusammensetzung zu bereiten, wie er in Nährlösungen ohne Fettgehalt gebräuchlich ist. Dies ist von besonderer Wichtigkeit, da schwer katabole Patienten, und gerade solche müssen häufig parenteral ernährt werden, einen erhöhten Bedarf an Kalium und Phosphat besitzen, das auf diese Weise dem Organismus zugeführt werden kann. Ebenso kann die erfindungsgemäß hergestellte Fettemulsion Nalon, Calcium-Ion, Mg-Ion und Zn-Ionen enthalten, sodaß auch der Bedarf an diesen Ionen gedeckt werden kann. Auch eine Tendenz zur acidotischen Stoffwechsellage kann durch entsprechende Dosierung von Acetation abgefangen werden.

Folgender Elektrolytgehalt hat sich pro Liter der erfindungsgemäßen Fettemulsion sowohl aus Stabilitätsgründen als auch vom medizinischen Standpunkt aus besonders bewährt:

| Na | 50 - 70 mmol |
| K | 20 - 30 mmol |
| Ca | 2 - 3 mmol |
| Mg | 2 - 5 mmol |
| Zn | 0,03 - 0,06 mmol |
| Cl | 40 - 70 mmol |
| Acetat | 15 - 25 mmol |
| Phosphat | 15 - 25 mmol, |

wobei Phosphat zweckmäßig in organischer Bindung z. B. in Form eines Glucose und/oder Fructosephosphates oder aber als Glycerophosphat zugesetzt werden kann.

Ebenso können im Rahmen der erfindungsgemäßen Verwendung sowohl die Menge als auch die Art der im Gemisch vorhandenen Aminosäuren den einzelnen Zustandsbildern von Patienten, die parenteral ernährt werden müssen, angepaßt werden. Das bedeutet, daß im Zuge des erfindungsgemäßen Verfahrens den Fettemulsionen nicht nur die essentiellen Aminosäuren einverleibt werden können, sondern auch jene nichtessentiellen Aminosäuren, die vor allem bei der Verabreichung solcher Nährlösungen an katabole Patienten oder solche im Coma nicht fehlen sollen. So ist man beispielsweise im Gegensatz zum Stand der Technik nicht mehr gezwungen, bei der parenteralen Ernährung mit Fett auf den Zusatz von Asparaginsäure, oder auch von der zu den glucoplastischen Aminosäuren zählenden Glutaminsäure, die also eine Glucosereserve darstellt, zu verzichten. Der Glutaminsäurezusatz hat ferner den Vorteil, daß diese Aminosäure ebenso wie Arginin, dessen Zusatz nun ebenfalls keine Grenzen mehr gesetzt sind, zu der Ammoniakentgiftung beiträgt, also beispielsweise im hepatischen Coma von Bedeutung ist.

Besonders bewährt haben sich Gesamtnährlösungen mit erfindungsgemäß hergestellten Fettemulsionen als Fettkomponente, die je 50 g emulgiertem Fett 5 - 7 g Phospholipid als Emulgator, 0,3 - 0,4 g Ubidecarenon, 50 - 150 g Zuckeralkohole, 50 - 150 mmol Elektrolyte sowie die folgenden Aminosäuren

| | |
|---|---|
| L-Isoleucin | 1 - 2,2 g |
| L-Leucin | 1,8 - 3,5 g |
| L-Lysin | 1,8 - 3 g |
| L-Methionin | 0,5 - 2 g |
| L-Phenylalanin | 1,5 - 2,3 g |
| L-Threonin | 1,2 - 2,0 g |
| L-Tryptophan | 0,4 - 0,9 g |
| L-Valin | 1,3 - 2,8 g |
| L-Arginin | 2,0 - 5,3 g |
| L-Histidin-Base | 1,4 - 2,3 g |
| L-Alanin | 3 - 7,1 g |
| L-Asparaginsäure | 0,5 - 1,0 g |
| L-Glutaminsäure | 1,3 - 2,0 g |
| Glycin | 1,7 - 4 g |
| L-Prolin | 2,5 - 4,5 g |
| L-Serin | 1,3 - 4,25 g |
| L-Ornithin | 0 - 0,8 g |
| L-Cystein | 0,1 - 0,4 g |
| L-Tyrosin | 0,4 - 1 g |

enthalten. Diese Lösung kann beispielsweise vorteilhaft als 3 - 7,5%ige Aminosäurelösung vorliegen, die dann bis zu 7,5 % Fett enthalten kann.

Es ist ferner auch möglich, den erfindungsgemäßen Gesamtnährlösungen ohne Störung der Stabilität Carnitin beizufügen, wenn z. B. eine Verbesserung der Fettverwertung erzielt werden soll. Dieser Zusatz bringt zusätzlich durch den gleichzeitigen Gehalt an Ubidecarenon den Vorteil mit sich, daß eine wesentlich höhere Fettclearance resultiert, eine Wirkung, die nur beide Substanzen gemeinsam zeigen. Meist hat sich pro 50 g Fett ein Zusatz von 0,5 -2,5 g L-Carnitin als solches oder in Form eines Hydrochlorids bewährt. Auch die an sich bekannte Zugabe von Fettsäuren mittlerer C-Atom-Anzahl zur Erhöhung der Stabilität ist möglich.

Beispiel 1:

In etwa 900 ml Wasser werden

| | |
|---|---|
| 1,050 g | L-Isoleucin |
| 1,800 g | L-Leucin |
| 1,950 g | L-Lysin |
| 0,570 g | L-Methionin |
| 1,500 g | L-Phenylalanin |
| 1,260 g | L-Threonin |
| 0,450 g | L-Trypthophan |
| 1,800 g | L-Valin |
| 2,400 g | L-Arginin |
| 1,800 g | L-Histidin als Base |
| 4,800 g | L-Alanin |
| 0,630 g | L-Asparaginsäure |
| 2,700 g | L-Glutaminsäure |
| 1,800 g | Glycin |
| 2,700 g | L-Prolin |
| 1,350 g | L-Serin |
| 0,750 g | L-Ornithin |
| 0,390 g | L-Cystein |
| 0,450 g | L-Tyrosin |

ferner

| | |
|---|---|
| 1,000 g | L-Carnitin |
| 100,000 g | Sorbit |
| 5,000 g | Soja-Lezithin |

und schließlich

| | |
|---|---|
| 60,0 mmol | Na |
| 30,0 mmol | K |
| 2,5 mmol | Ca |
| 3,75 mmol | Mg |
| 0,0044 mmol | Zn |
| 62,6 mmol | Cl |
| 20,0 mmol | Acetat |
| 20,0 mmol | Phosphat |

in Form von Glycerophosphat gelöst, in der Lösung durch Zugabe von NaOH ein pH-Wert von 7,0 eingestellt, und diese auf 75°C vorgewärmt, und in einen Mischer eingetragen, der einen nach dem Scherkopfprinzip arbeitenden Homogenisierteil enthält. Nach Begasen mit sterilem Stickstoff wird eine Lösung von 0,375 g Ubidecarenon in 50 g Sojaöl in den auf 5000 U/Minute laufenden Mischer eingetragen, und 20 - 30 Minuten emulgiert, bis eine Tröpfchenfeinheit von 2 - 3 µm resultiert. Daraufhin wird diese Voremulsion in einen 2-stufig arbeitenden Hochdruckhomogenisator eingebracht, der mit sterilem Stickstoff begast wird, in dem bei viermaligem Durchgang bei einem Druck von 400 bar ein Homogenat mit einer durchschnittlichen Tröpfchengröße von unter 1 µm erzeugt wird.

Zur Entfernung ev. vorhandener größerer Restbestandteile wird die Emulsion durch ein 3 µm-Membranfilter filtriert, nach Auffüllen mit sterilem Wasser auf 1 l abgefüllt und das abgefüllte Produkt bei 80°C im Rotationsautoklaven sterilisiert.

Man erhält so eine 3 %ige transferadaptierte Aminosäurelösung mit einem Fettgehalt von 50 g/l.

Beispiel 2:

Aus

| | |
|---|---|
| 2,200 g | L-Isoleucin |
| 3,400 g | L-Leucin |
| 2,880 g | L-Lysin |
| 2,000 g | L-Methionin |
| 2,300 g | L-Phenylalamin |
| 2,000 g | L-Threonin |
| 0,900 g | L-Tryptophan |
| 2,800 g | L-Valin |
| 5,250 g | L-Arginin |
| 2,250 g | L-Histidin |
| 7,100 g | L-Alanin |
| 1,000 g | L-Asparaginsäure |
| 0,250 g | L-Cystein |
| 2,000 g | L-Glutaminsäure |
| 4,000 g | Glycin |
| 4,500 g | L-Prolin |
| 4,250 g | L-Serin |
| 1,000 g | L-Tyrosin |
| 60 mmol | Na |
| 30 mmol | K |
| 2,5 mmol | Ca |
| 3,75 mmol | Mg |
| 0,044 mmol | Zn |
| 62,6 mmol | Cl |
| 20,0 mmol | Acetat |
| 20,0 mmol | Phosphat als Glycerophosphat |
| 100,000 g | Sorbit |
| 6,000 g | Eilezithin |

sowie

| | |
|---|---|
| 0,5 g | Ubidecarenon und |
| 50 g | Sojaöl |

sowie der nötigen Menge Wasser werden analog zu Beispiel 1 1000 ml einer 5 %igen Aminosäurelösung mit einem Gehalt an 50 g Fett bereitet.

Die Zusammensetzung der Aminosäurelösung entspricht dem Kartoffel-Ei-Muster und ist besonders zur Verabreichung an Patienten mit Katabolie geeignet.

8

**Patentansprüche**

1. Verfahren zur Herstellung einer stabilen wäßrigen Fettemulsion zur Verwendung in Lösungen zur parenteralen Ernährung, durch Emulgieren des Fettes unter hohem Druck und unter Einsatz von Phospholipiden, insbesondere Lezithinen, als Emulgator, dadurch gekennzeichnet, daß neben dem als Emulgator dienenden Phospholipid Ubidecarenon als Hilfsemulgator in einem Gewichtsverhältnis von Phospholipid zu Ubidecarenon von 150 : 1 bis 7,5 : 1 eingesetzt wird und das Gewicht der beiden Emulgatoren zusammen zum Gewicht der zu emulgierenden Fettmenge im Verhältnis 1 : 7 bis 1 : 30 steht.

2. Verfahren gemäß Anspruch 1, dadurch gekennzeichnet, daß das als Emulgator dienende Phospholipid Sojalezithin oder Fraktionen desselben ist.

3. Verfahren nach den Ansprüchen 1 oder 2, dadurch gekennzeichnet, daß das Gewichtsverhältnis von Phospholipid zu Ubidecarenon von 30 : 1 bis 10 : 1 beträgt.

4. Verfahren nach den Ansprüchen 1 bis 3, dadurch gekennzeichnet, daß das als Fett Sojaöl eingesetzt wird und die Summe der Gewichte an Phospholipid und Ubidecarenon zum Gewicht des Fettes in einem Verhältnis von 1 : 7,5 bis 1 : 15 steht.

5. Verfahren nach einem der Ansprüche 1 bis 4, dadurch gekennzeichnet, daß ein Gemisch des zu emulgierenden Fettes mit dem Ubidecarenon in eine vorgelegte wäßrige Mischung, die das Phospholipid sowie gegebenenfalls vorhandene übrige Bestandteile enthält, unter hefti gem Rühren eingetragen wird, worauf die so erhaltene Voremulsion der an sich bekannten Hochdruckemulgierung bei Drücken von 400 - 600 bar bis zur Erzielung einer Tröpfchenfeinheit von durchschnittlich 1 µm unterworfen wird.

6. Verwendung einer gemäß einem der Ansprüche 1 bis 5 hergestellten Fettemulsion als Fettkomponente in parenteral zu verabreichenden Gesamtnährlösungen.

7. Verwendung nach Anspruch 6, dadurch gekennzeichnet, daß die Gesamtnährlösungen Elektrolyte in für fettfreie Gesamtnährlösungen üblicher Zusammensetzung und Menge enthalten.

8. Verwendung nach Anspruch 6 oder 7, dadurch gekennzeichnet, daß die Gesamtnährlösungen neben den essentiellen Aminosäuren Arginin, Glutaminsäure und/oder Asparaginsäure sowie gewünschtenfalls weitere nicht essentielle Aminosäuren enthalten.

9. Verwendung nach einem der Ansprüche 6 bis 8, dadurch gekennzeichnet, daß die Gesamtnährlösungen auf 50 g Fett 0,5 bis 2,5 Carnitin enthalten.

10. Verwendung einer stabilen, wäßrigen Fettemulsion, die durch Emulgieren des Fettes unter hohem Druck und unter Einsatz von Phospholipiden, insbesondere Lezithinen, hergestellt wurde, als Fettkomponente in parenteral zu verabreichenden Gesamtnährlösungen, dadurch gekennzeichnet, daß bei der Bereitung der Fettemulsion Ubidecarenon als Hilfsemulgator diente und die Gesamtnährlösungen auf je 50 g der emulgierten Fettkomponente 5 bis 7 g Phospholipid als Emulgator, 0,3 bis 0,4 g Ubidecarenon als Hilfsemulgator, sowie 50 bis 150 g Zuckeralkohole, 50 bis 150 mmol Elekrolyte sowie die folgenden Aminosäuren

| L-Isoleucin | 1 | - | 2,2 | g |
|---|---|---|---|---|
| L-Leucin | 1,8 | - | 3,5 | g |
| L-Lysin | 1,8 | - | 3 | g |
| L-Methionin | 0,5 | - | 2 | g |
| L-Phenylalanin | 1,5 | - | 2,3 | g |
| L-Threonin | 1,2 | - | 2,0 | g |
| L-Tryptophan | 0,4 | - | 0,9 | g |
| L-Valin | 1,3 | - | 2,8 | g |
| L-Arginin | 2,0 | - | 5,3 | g |
| L-Histidin-Base | 1,4 | - | 2,3 | g |
| L-Alanin | 3 | - | 7,1 | g |
| L-Asparaginsäure | 0,5 | - | 1,0 | g |
| L-Glutaminsäure | 1,3 | - | 2,0 | g |
| Glycin | 1,7 | - | 4,0 | g |
| L-Prolin | 2,5 | - | 4,5 | g |
| L-Serin | 1,3 | - | 4,25 | g |
| L-Ornithin | 0 | - | 0,8 | g |
| L-Cystein | 0,1 | - | 0,4 | g |
| L-Tyrosin | 0,4 | - | 1 | g |

enthalten.

**Claims**

1. A process for the preparation of a stable aqeous fat emulsion for use in solutions for parenteral alimentation, by emulsifying the fat under high pressure and with the use of phospholipids, in particular lecithins, as the emulsifier, wherein, in addition to the phospholipid used as the emulsifier, ubidecarenone is used as the auxiliary emulsifier in a weight ratio of phospholipid to ubidecarenone of from 150:1 to 7.5:1, and the weight of the two emulsifiers together is in a ratio to the weight of the amount of fat to be emulsified of from 1:7 to 1:30.

2. A process as claimed in claim 1, wherein the phospholipid used as the emulsifier is soya lecithin or fractions thereof.

3. A process as claimed in either of claims 1 and 2, wherein the weight ratio of phospholipid to ubidecarenone is from 30:1 to 10:1.

4. A process as claimed in any of claims 1 to 3, wherein soya oil is used as the fat and the sum of the weights of phospholipid and ubidecarenone is in a ratio to the weight of the fat of from 1:7.5 to 1:15.

5. A process as claimed in any of claims 1 to 4, wherein a mixture of the fat to be emulsified with the ubidecarenone is introduced, with vigorous stirring, into a previously introduced aqueous mixture containing the phospholipid with or without customary constituents, and the pre-emulsion thus obtained is then subjected to a conventional high pressure emulsification at from 400 to 600 bar until a droplet fineness of on average 1 μm is achieved.

6. The use of a fat emulsion prepared as claimed in any of claims 1 to 5 as a fat component in total nutrient solutions for parenteral administration.

7. The use as claimed in claim 6, wherein the total nutrient solutions contain electrolytes in the composition and amount customary for fat-free total nutrient solutions.

8. The use as claimed in either of claims 6 and 7, wherein the total nutrient solutions contain, in addition to the essential amino acids, arginine, glutamic acid or aspartic acid and, if required, further nonessential amino acids.

9. The use as claimed in any of claims 6 to 8, wherein the total nutrient solutions contain 0.5–2.5 g of carnitine per 50 g of fat.

10. The use of a stable, aqueous fat emulsion, prepared by emulsifying the fat under high pressure and with the use of phospholipids, in particular lecithins, as a fat component in total nutrient solutions for parenteral administration, wherein ubidecarenone was used as the auxiliary emulsifier in the preparation of the fat emulsion and the total nutrient solutions contain, per 50 g of the emulsified fat component, 5–7 g of phospholipid as the emulsifier, 0.3-0.4 g of ubidecarenone as the auxiliary emulsifier and 50–150 g of sugar alcohols, 50-150 mmol of electrolytes and the following amino acids

| | | |
|---|---|---|
| L-isoleucine | 1 - 2.2 | g |
| L-leucine | 1.8 - 3.5 | g |
| L-lysine | 1.8 - 3 | g |
| L-methionine | 0.5 - 2 | g |
| L-phenylalanine | 1.5 - 2.3 | g |
| L-threonine | 1.2 - 2.0 | g |
| L-tryptophan | 0.4 - 0.9 | g |
| L-valine | 1.3 - 2.8 | g |
| L-arginine | 2.0 - 5.3 | g |
| L-histidine base | 1.4 - 2.3 | g |
| L-alanine | 3 - 7.1 | g |
| L-aspartic acid | 0.5 - 1.0 | g |
| L-glutamic acid | 1.3 - 2.0 | g |
| glycine | 1.7 - 4 | g |
| L-proline | 2.5 - 4.5 | g |
| L-serine | 1.3 - 4.25 | g |
| L-ornithine | 0 - 0.8 | g |
| L-cysteine | 0.1 - 0.4 | g |
| L-tyrosine | 0.4 - 1 | g. |

**Revendications**

1. Procédé de préparation d'un émulsion grasse aqueuse, stable, pour utilisation dans des solutions destinées à l'alimentation parentérale, par émulsion de la graisse sous pression élevée et avec incorporation de phospholipides, en particulier de lécithines comme émulsionnant, caractérisé par le fait qu'à côté du phospholipide servant d'émulsionnant, on introduit de l'ubidécarénone comme émulsionnant auxiliaire, en un rapport en poids du phospholipide à l'ubidécarénone de 150/1 à 7,5/1 et le poids des deux émulsionnants ensemble est, par rapport au poids de la dose grasse à émulsionner, de 1/7 à 1/30.

2. Procédé selon la revendication 1, caractérisé par le fait que le phospholipide servant d'émulsionnant est la lécithine du soja ou des fractions de celle-ci.

3. Procédé selon les revendications 1 ou 2, caractérisé par le fait que le rapport en poids de la phospholipide à l'ubidécarénone est de 30/1 à 10/1.

4. Procédé selon les revendications 1 à 3, caractérisé par le fait que l'on introduit comme graisse de l'huile de soja et la somme des poids de phospholipide et ubidécarénone est en un rapport de 1/7, 5 à 1/15 par rapport au poids de la graisse.

5. Procédé selon l'une des revendications 1 à 4, caractérisé par le fait qu'on introduit, sous forte agitation, un mélange de la graisse à émulsionner avec la ubidécarénone, dans un mélange aqueux préparé, qui contient le phospholipide ainsi que des constituants restants éventuellement présents, après quoi on soumet la pré-émulsion obtenue à l'émulsionnement haute pression, connu en soi, à des pressions de 400–600 bar, jusqu'à obtention d'une finesse de gouttelettes de 1 μm en moyenne.

6. utilisation d'une émulsion grasse, préparée selon l'une des revendications 1 à 5, comme composante grasse dans les solutions d'alimentation générale à administrer par voie parentérale.

7. utilisation, selon la revendication 6, caractérisée par le fait que les solutions d'alimentation générale contiennent des électrolytes en composition et quantité usuelles pour des solutions d'alimentation générale sans graisse.

8. Utilisation, selon la revendication 6 ou 7, caractérisée par le fait que les solutions d'alimentation générale contiennent: outre les amino-acides, essentiels, de l'arginine, de l'acide glutamique et/ou de l'aci-

11

de aspariginique ainsi que, si on le souhaite, d'autres acides aminés non essentiels.

9. Utilisation selon l'une des revendications 6 à 8, caractérisée par le fait que les solutions d'alimentation générale contiennent, sur 50 g de graisse, 0,5 à 2,5 g de carnitine.

10. Uilisation d'une émulsion grasse, aqueuse, stable, qui a été préparée par émulsionnement de la graisse sous pression élevée et avec incorporation de phospholipides, en particulier de lécithines, comme composante grasse dans les solutions d'alimentation générale à administrer par voie parentérale, caractérisée par le fait que, pour la préparation de l'émulsion grasse, l'ubidécarénone à servi d'émulsionnant auxiliaire et les solutions d'alimentation générale contiennent, pour 50 g du composant gras émulsionné, 5 à 7 g de phospholipide comme émulsionnant, 0,3 à 0,4 g de ubidécarénone comme émulsionnant auxiliaire, ainsi que 50 à 150 g d'alcools sucrés, 50 à 150 moles d'électrolytes ainsi que les amino-acides suivants:

| L-isoleucine | 1 | - | 2,2 | g |
|---|---|---|---|---|
| L-leucine | 1,8 | - | 3,5 | g |
| L-lysine | 1,8 | - | 3 | g |
| L-méthionine | 0,5 | - | 2 | g |
| L-phénylalanine | 1,5 | - | 2,3 | g |
| L-thréonine | 1,2 | - | 2,0 | g |
| L-tryptophane | 0,4 | - | 0,9 | g |
| L-valine | 1,3 | - | 2,8 | g |
| L-arginine | 2,0 | - | 5,3 | g |
| L-histidine-base | 1,4 | - | 2,3 | g |
| L-alanine | 3 | - | 7,1 | g |
| acide L-asparaginique | 0,5 | - | 1,0 | g |
| acide L-glutaminique | 1,3 | - | 2,0 | g |
| glycine | 1,7 | - | 4,0 | g |
| L-prolyne | 2,5 | - | 4,5 | g |
| L-serine | 1,3 | - | 4,25 | g |
| L-ornithine | 0 | - | 0,8 | g |
| L-cystéine | 0,1 | - | 0,4 | g |
| L-tyrosine | 0,4 | - | 1 | g |